# EUROPEAN PATENT SPECIFICATION

(11) **EP 3 752 831 B1**
(45) Date of publication and mention of the grant of the patent: **25.03.2026**
(21) Application number: 19707068.3
(22) Date of filing: 13.02.2019
(51) Int. Cl.: G01N 33/50, A61B 5/1172

(54) **A METHOD OF ANALYSING A SKIN-PRINT**
VERFAHREN ZUR ANALYSE EINES HAUTABDRUCKS
PROCÉDÉ D'ANALYSE D'EMPREINTE CUTANÉE

(30) Priority: 13.02.2018 GB 201802356
(43) Date of publication of application: 23.12.2020
(73) Proprietor: Intelligent Fingerprinting Limited, Cambridgeshire CB24 9NG (GB)
(72) Inventor: HUDSON, Mark, Cambridgeshire CB24 9NG (GB); WILSON, Paul, Cambridgeshire CB24 9NG (GB); RUSSELL, David Andrew, Cambridgeshire CB24 9NG (GB); WALKER, Jeremy Nigel Burgess, Cambridgeshire CB24 9NG (GB)
(74) Representative: Boult Wade Tennant LLP
(86) International application number: PCT/GB2019/050385
(87) International publication number: WO 2019/158918

(56) References cited:
- WO-A1-2016/135497
- WO-A2-2006/073450
- POTCOAVA M C ET AL: "Fingerprint biometry applications of digital holography and low-coherence interferography", APPLIED OPTICS, OPTICAL SOCIETY OF AMERICA, WASHINGTON, DC; US, vol. 48, no. 34, 1 December 2009 (2009-12-01), pages H9 - H15, XP001550252, ISSN: 0003-6935, DOI: 10.1364/AO.48.0000H9
- ARCHER N E ET AL: "Changes in the lipid composition of latent fingerprint residue with time after deposition on a surface", FORENSIC SCIENCE INTERNATIONAL, ELSEVIER B.V, AMSTERDAM, NL, vol. 154, no. 2-3, 25 November 2005 (2005-11-25), pages 224 - 239, XP027757791, ISSN: 0379-0738, [retrieved on 20051125]

## Description

### BACKGROUND

An impression left by the friction ridges of human skin, such as the skin of a human finger, contains information regarding the identity of the human. It is widely known that the appearance of the impression of the human finger, known as a fingerprint, is unique to each human and may be used to confirm the identity of the human. The appearance of the impression of the skin of other human body parts may also be unique to each human and so may also be used to confirm the identity of the human. Impressions of human skin, including but not limited to the skin of the human finger, may be called skin-prints.

In the present disclosure, it should be noted that the term skin-print is used to refer to the residue of a deposited skin-print rather than to the constituents of the skin-print residue when present on the human skin. The skin-print contains sweat which may include both eccrine sweat and sebaceous sweat.

In addition to the appearance of the impression left by human skin, the impression may contain chemical species which themselves may be detected in order to obtain further information.

For example, when a human intakes a substance (e.g. by ingestion, inhalation, injection or any other means) the substance may be metabolised by the human body giving rise to secondary substances known as metabolites. The presence of a particular metabolite can be indicative of a specific intake substance. The intake substance and/or metabolites may be present in sweat and, as such, may be left behind in a skin-print, e.g. a latent/residual fingerprint. Detection of such metabolites in a skin-print can be used as a non-invasive method of testing for recent lifestyle activity such as (but not limited to) drug use, or compliance with a pharmaceutical or therapeutic treatment regime. Other applications include drug rehabilitation, criminal justice and research into latent fingerprints.

Importantly, the taking of a skin-print is much simpler than obtaining other body fluids such as blood, saliva and urine, and is more feasible in a wider range of situations. Not only this but since the appearance of the skin-print itself provides confirmation of the identity of the person providing the skin-print, there can be greater certainty that the substance or substances in the skin-print are associated with the individual. This is because substitution of a skin-print, particularly a fingerprint, is immediately identifiable from appearance whereas substitution of, for example, urine, is not immediately identifiable from appearance. As such, testing for one or more substances in a skin-print provides a direct link between the one or more substances and the identity of the human providing the skin-print.

The applicant has demonstrated various techniques for chemical analysis of skin-prints, including the use of mass spectrometry, for example paper spray mass spectrometry. The applicant has also developed a lateral flow skin-print analysis technique as described in WO 2016/012812, published 28 January 2016.

Obtaining an indication of a quantity, for example mass (or possibly volume), of a metabolite present in a skin-print sample may be more informative if given as a measure relative to quantity, for example by volume (or possibly by mass), of skin-print. This may be particularly applicable in situations where an acceptable threshold (measured in, for example, mass of analyte per unit volume of skin-print) is defined, for example by an independent standards agency.

For example, a relatively small amount of metabolite present in a relatively large volume/mass of skin-print may be less significant than a relatively larger amount of metabolite present in only a relatively small volume/mass of skin-print.

WO 2016/135497 discloses a device for receiving and analysing a sample, wherein the analysing involves use of a solution. The device comprises: a sample receiving portion for receiving a sample to be analysed; and a solution capsule having a sealed configuration in which the solution capsule is sealed and a release configuration in which contents of the solution capsule are released via a flow path that provides fluid communication between the solution capsule and the sample receiving portion. The device further comprises a bistable release mechanism comprising an actuator wherein the bistable release mechanism releases only in the event that a force applied to the actuator reaches a threshold force and wherein actuation of the actuator results in one-way conversion of the solution capsule from the sealed configuration into the release configuration.

WO2006/073450 discloses an optical fingerprinting method extracts high quality latent fingerprints from a surface without any invasive chemical or physical contact with the examined object, and requires no cooperation of the subject. Rather than employing extraneous material, the optical properties of the latent fingerprint are used to generate one or more images with sufficient contrast to distinguish the latent fingerprint or some other deformation in the surface. The system includes a light source oriented to apply light at an angle of incidence to the surface at the position to be examined for the latent fingerprint or deformation, a camera oriented to receive light specularly and diffusely reflected from the surface and/or by the fingerprint or deformation on the surface, and a processor that performs the computation for digital contrast enhancement and/or reprojection of the recovered fingerprint image to a frontal view if necessary. The technique uses optical polarization properties to enhance the images by placing a linearly polarized filter(s) in front of the observing camera. At least two pictures of the same scene with the same lighting and view angle arrangement are taken whreby each of the pictures differ only in that the orientations of the polarization filter are different. At least two light polarization parameters for each pixel are computed from the two or more images taken with different polarizer orientations. An image with each pixel value representing the value of one of the polarization parameters or a function of the polarization parameters is generated and displayed, with some digital contrast enhancement and/or reprojection applied. The hidden latent fingerprint pattern is revealed in at least one such image with the interfering background pattern significantly suppressed.

Potcoava M C et al: "Fingerprint biometry applications of digital holography and low-coherence interferography", Applied Optics, Optical Society of America, Washington, DC; US, vol. 48, no. 34, 1 December 2009, pages H9-H15, ISSN: 0003-6935 discloses holographic and interographic methods for two- and three-dimensional imaging of fingerprints.

Archer N E et al: "Changes in the lipid composition of latent fingerprint residue with time after deposition on a surface", Forensic Science International, Elsevier B.V, Amsterdam, NL, vol. 154, no.2-3, 25 November 2005, pages 224-239, ISSN: 0379-0738 discusses fingerprint chemistry, particularly composition of a latent fingerprint at the time it is deposited, and the chemical changes in lipid components that occur over time.

Accordingly, a need exists for a technique to determine a quantity of skin-print deposited in order to be able to determine an amount of analyte per unit of deposited skin-print.

### STATEMENTS OF INVENTION

Against this background, there is provided a method for analysing a skin-print, the method comprising:
receiving a skin-print on a substrate;
performing a quantity test to obtain a quantity score for the skin-print , wherein performing the quantity test to obtain a quantity score for the skin-print comprises determining a volume of skin-print received on the substrate using an optical technique comprising emitting incident light onto the skin-print in order to determine a proportion of incident light that is optically influenced by the skin-print as a proportion of a total of the incident light;
performing a chemical analysis of the skin-print to detect for the presence of one or more chemical species and thereby provide a chemical test result;
storing or transmitting the chemical test result together with the quantity score to enable calculation of a contextualised chemical test result relative to the volume of skin-print.

Preferably, the chemical analysis provides a mass of one or more analytes under test. Thus, the method may provide a result in terms of unit mass of analyte per unit volume of skin-print.

### BRIEF DESCRIPTION OF THE DRAWINGS

Figure 1 shows a flow chart of a method of analysing a skin-print in accordance with the disclosure;
Figures 2a, 2b and 2c show an apparatus that may be employed to obtain a quantity score in accordance with an embodiment of the method of the disclosure;
Figure 3 shows a flow chart illustrating a process by which the apparatus of Figures 2a, 2b and 2c may be used to determine a quantity score and a volume of a skin-print in accordance with the present disclosure; and

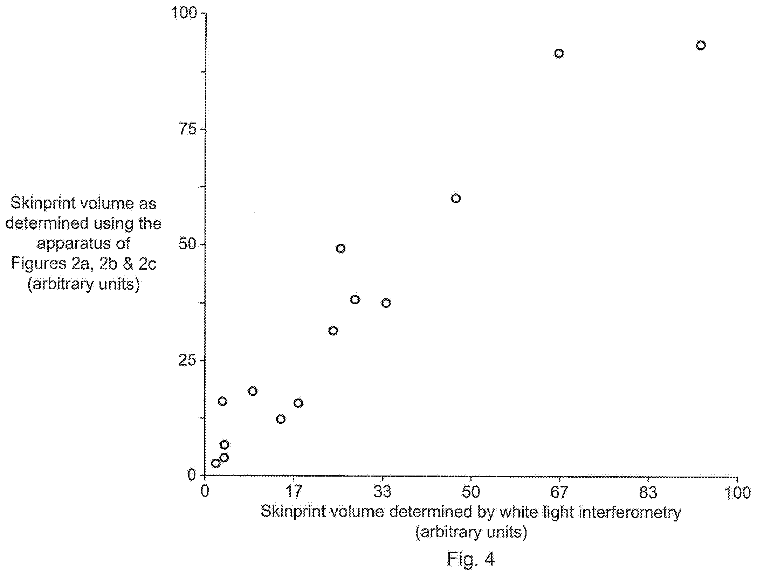

Figure 4 shows a plot of the relationship between volume of skin-print as determined using an apparatus of the arrangement shown in Figures 2a, 2b and 2c and volume determined using a white light interferometry technique.

### DETAILED DESCRIPTION

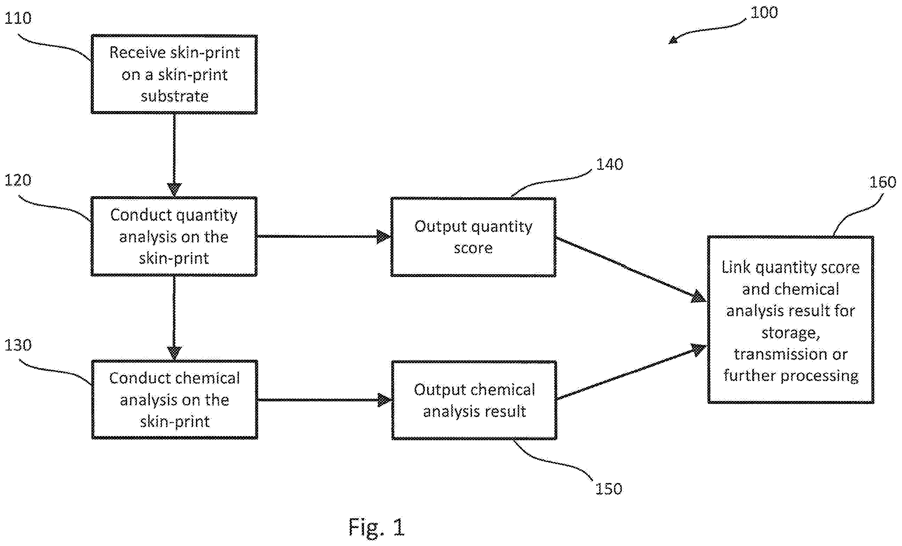

The present disclosure relates to a method of analysing a skin-print deposited on a substrate. A high level representation of the method is shown in the flow chart of Figure 1.

The method 100 involves receiving a skin-print on a substrate 110, obtaining a quantity score for the skin-print 120 and obtaining results of a chemical analysis of the skin-print 130 so as to allow the chemical test result to be contextualised relative to the quantity of skin-print.

The disclosure envisages a wide range of chemical analysis techniques and a wide range of quantity analysis techniques. Accordingly, the specific embodiments of chemical analysis technique and quantity analysis technique discussed herein should be interpreted as examples of a range of available options that fall within the scope of the claimed invention.

The term "quantity test" encompasses any technique that provides an objective measure that is, or is related to, the quantity of skin-print present on the substrate under test. One objective quantity test of a skin-print may involve determining mass of the skin-print. Another objective quantity test of a skin-print may involve determining volume of the skin-print.

It will be appreciated that the quantity of material deposited in a skin-print sample is likely to be modest. Accordingly, the quantity may not be straightforwardly measured. To measure mass or volume of skin-print directly and in a non-destructive manner may require expensive scientific instrumentation. This may not be possible in the field. For example, high precision balances that might be used to measure the mass of a substrate both before and after a skin-print has been deposited may be both expensive and require controlled environments such as anti-vibration tables and cleanroom conditions to avoid effects of vibrational noise and contamination. In order to obviate the need for such equipment, the applicant has developed techniques for determining a quantity score for a skin-print that rely upon determining mass/volume (to within a known level of uncertainty) by directly measuring other attributes of the skin-print.

Some of these techniques involve performing an optical analysis on the skin-print in order to determine the degree to which the optical properties of a substrate are altered by virtue of a skin-print deposited upon the substrate. The measurement of such optical properties has been found by the Applicant to relate to the measure of mass or volume of skin-print.

In an example falling outside the scope of the claims, wherein the quantity score of a skin-print is related to the mass of the skin-print, the quantity score may simply be the mass of the skin-print. Or it may be proportional to the mass of the skin-print. Or it may be a function of the mass of the skin-print. Any approach by which the mass of the skin-print may be measured or derived may be appropriate. Such examples involving mass fall outside the scope of the claims.

Similarly, in an example wherein the quantity score of a skin-print is related to the volume of the skin-print, the quantity score may simply be the volume of the skin-print. Or it may be proportional to the volume of the skin-print. Or it may be a function of the volume of the skin-print. Any approach by which the volume of the skin-print may be measured or derived may be appropriate. In order to fall within the scope of the claims, however, the volume of skin-print is determined using the specified optical technique.

It should also be understood that other characteristics of the skin-print (instead of or in addition to mass/volume) may contribute to the objective assessment of skin-print quantity.

In accordance with the claims, performing a quantity test to obtain a quantity score for the skin-print comprises determining a volume of skin-print received on the substrate using an optical technique comprising emitting incident light onto the skin-print in order to determine a proportion of incident light that is optically influenced by the skin-print as a proportion of a total of the incident light. Techniques discussed herein that do not involve this requirement do not fall within the scope of the claims.

The specific examples of chemical analysis disclosed herein should be understood as merely exemplary rather than prescriptive.

### Non-exhaustive list of techniques for obtaining a quantity score

A quantity score may be obtained using a variety of different techniques, including the following. Not all of these techniques fall within the scope of the claims. To fall within the scope of the claims, performing a quantity test to obtain a quantity score for the skin-print comprises determining a volume of skin-print received on the substrate using an optical technique comprising emitting incident light onto the skin-print in order to determine a proportion of incident light that is optically influenced by the skin-print as a proportion of a total of the incident light.

In one example falling outside the scope of the claims, interferometry may be used to establish the skin-print's topography in order to determine skin-print volume. The interferometry technique may be or comprise white light interferometry.

Detection of the influence of a skin print on the passage or propagation of electromagnetic radiation may be used to determine mass or volume, perhaps by calibration with another technique such as white light interferometry.

In another example falling outside the scope of the claims, surface plasmon resonance imaging may be used, where the substrate material allows, to establish a topographical map of the skin-print from which volume can be determined.

In an embodiment falling within the scope of the claims, obtaining a quantity score involves emitting electromagnetic radiation towards the substrate and analysing how the electromagnetic radiation is influenced by the presence of a skin-print and the amount of skin-print deposited. A reflection of the electromagnetic radiation from the substrate may then be captured, recorded and analysed. The electromagnetic radiation may fall within the visible spectrum (either broadband spectrum or a specific wavelength) and the technique may involve determining the effect of the skin-print on the visible spectrum radiation.

In a more specific example, discussed in more detail below, a technique involves use of an optical waveguide having a fingerprint receiving region. A skin-print deposited on the fingerprint receiving region acts as a coupling device that selectively permits coupling of the optical radiation into/out of the waveguide dependent upon the amount of skin-print deposited.

In an alternative optical approach falling outside the scope of the claims, an optical image may be taken of a deposited skin-print which is analysed using image processing software in order to provide an objective measure of the amount of skin-print deposited.

In a further alternative optical approach falling outside the scope of the claims, an optical image may be taken of light reflected off a deposited skin-print and analysed using image processing software in order to provide an objective measure of the amount of skin-print deposited.

Still further examples of techniques that may be adopted as part of a quantity scoring process include (but are not limited to) the following:
- Optical coherence tomography;
- Confocal microscopy;
- Atomic force microscopy;
- 3D laser mapping;
- Ellipsometry;
- Scanning tunnelling microscopy (STM); and
- Image analysis following staining with a developer agent such as Ninhydrin; and
- Image analysis following staining with a detection agent such as Nile red.

If any of these techniques is adopted without the use of an optical technique comprising emitting incident light onto the skin-print in order to determine a proportion of incident light that is optically influenced by the skin-print as a proportion of a total of the incident light, then such a technique would not fall within the scope of the appended claims.

It is possible to implement many of these techniques on the substrate both before and after deposition of the skin-print in order to provide a reference or control with which a comparison may be made.

It is also envisaged that calibration of one or more quantity score techniques may be appropriate and/or indeed necessary. For example, in one scenario, an optical based quantity score technique may be calibrated using a technique based on white light interferometry. White light interferometry itself may provide a more direct route to quantity but may require a longer time and more complex apparatus. By contrast, the optical based techniques may be quicker and require less complicated apparatus but may provide a less direct route to quantity. Calibrating the optical based technique with white light interferometry may provide for increased confidence in quantity scores provided by the optical based technique and may be more amenable to calibration themselves using metrology standards.

Depending upon the specific combination of quantity analysis technique and chemical analysis technique, it may be that the order in which the techniques are adopted is important. This may particularly be the case if one technique might prejudice the outcome of the other. However, where there is no such potential prejudice, it may be that the quantity analysis and the chemical analysis are carried out in either order.

### Non-exhaustive list of chemical analysis techniques

A range of chemical analysis techniques falls within the scope of the disclosure.

One possibility is to use mass spectrometry techniques including:
- liquid chromatography-tandem mass spectrometry (LC-MS-MS);
- gas chromatography-tandem mass spectrometry (GC-MS-MS);
- matrix assisted laser desorption/ionisation time of flight mass spectrometry (MALDI-TOF MS);
- positional mapping mass spectrometry;
- paper spray mass spectrometry.

Raman Spectroscopy is still a further alternative chemical analysis technique.

Further alternatives include chromatography techniques including thin layer chromatography.

Further possibilities for chemical analysis techniques include skin-print development techniques including those employing substances such as Ninhydrin. This may be in combination with a separation method (e.g. thin film chromatography) whereby the chemical components are separated and then stained in order to aid in visualisation.

Further options include lateral flow analysis techniques such as those disclosed in the Applicant's previous patent publications including in application number PCT/GB2015/052157 published as WO 2016/012812.

In short, the disclosure envisages any appropriate chemical analysis technique from which one or more chemical constituents may be detected together with an indication of quantity of the chemical constituent.

Whatever chemical analysis technique is adopted, it may be that the chemical test result provided by it is in the form of a numerical output. In one example, the numerical output may be in the form of a precise mass of analyte in question. In another example, the numerical output may be a numerical indication that the mass of analyte falls within a particular mass range. In either of these examples, or in other cases, in order to contextualise the chemical test result, the result may be compared to the quantity score in order to provide a contextualised result.

The contextualised result may then be used to inform the intended assessment which may, for example, include whether or not the skin-print provides evidence of use of a particular drug or, for example, whether or not the skin-print provides evidence of complicity with a drug dosage regime.

The contextualised result may be compared to an independently determined threshold. One example would be an independently specified threshold at which drug use is considered clearly demonstrated. This might be measured in unit mass of drug metabolite per unit volume of skin-print.

It may be necessary to calibrate the quantity score with a particular mass/volume or other metric.

In some circumstances it may be that a quantity analysis is performed at a different time and location from a chemical analysis technique. For example, it may be that the quantity analysis technique (perhaps a preliminary quantity analysis technique that precedes a secondary quantity analysis technique) is performed using portable apparatus that is available in the field and that the chemical analysis technique involves apparatus that is perhaps not portable or perhaps not available in the field. In this scenario, as in many others, it may be important that the quantity score obtained in the field using the quantity analysis technique is connected to the sample in question such that the result of the chemical analysis score is reconciled with the exact same sample to which the quantity score relates. Accordingly, it may be that the sample substrate or an ancillary to the sample substrate has a unique identifier. The unique identifier could use any technique by which the sample substrate could be uniquely identified. It may comprise some form of tamper evident features in order to provide evidence of an attempt to disguise the provenance of the sample.

In any event, even where quantity analysis technique and chemical analysis technique are carried out one immediately after the other (or even simultaneously), being confident that the results of each technique on a particular sample are linked to one another may be particularly important. Thus, use of a unique identifier to which the results are linked has a very broad range of applications.

While the unique identifier might involve one or more of any number of unique identifier features known to the skilled person, examples of such identifiers may include an RFID tag, a QR code, a bar code (perhaps engraved into the substrate) or other tags, whether electronic, mechanical, optical biological or any other suitable means for providing unique identification.

The unique identifier may in some examples also possess properties that allow it to receive and store information on the provenance of the sample or its quantity score or its identity, for example where a suitable identification algorithm is used in the analysis instrument to determine the identity of the sample donor. This might comprise a fingerprint matching algorithm.

### Detailed example of an optical analysis technique for determining a quantity score that falls within the scope of the claims

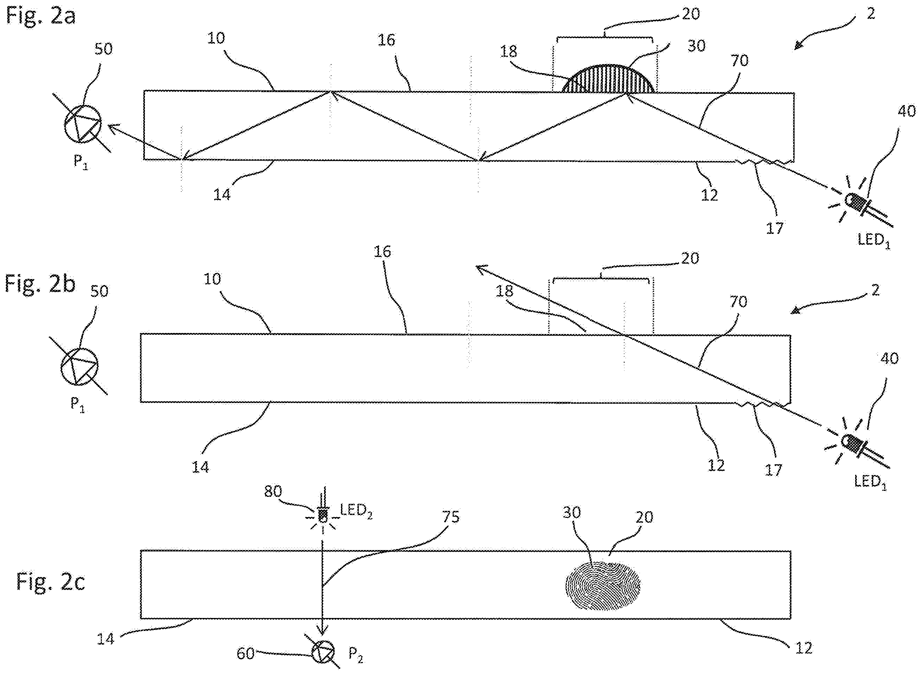

The Applicant has developed an optical analysis method and apparatus that may be used for determining a quantity score in accordance with the present disclosure. Figures 2a, 2b and 2c show an example of such apparatus 200 for determining an objective measure of a quantity of skin-print deposited on a fingerprint receiving region 20 of a planar non-porous substrate. Figures 2a and 2b show the apparatus from a side-on view. Figure 2c shows the apparatus from above.

The fingerprint receiving region 20 may provide a fixed area onto which a skinprint may be applied in order to increase consistency of area of skinprints between donors. The fixed area may be smaller than the average skinprint area. This may also have advantages for consistency if the same subject provides multiple prints, perhaps for different purposes.

The apparatus 200 comprises primary and secondary electromagnetic radiation sources 40, 80; a translucent waveguide 10 having a first end 12 and a second end 14; and first and second photodiodes 50, 60 configured to output a photodiode signal indicative of electromagnetic radiation detected by the first and second photodiodes 50, 60. The first photodiode 50 is configured to detect electromagnetic radiation emitted by the primary electromagnetic radiation source 40. The second photodiode 60 is configured to detect electromagnetic radiation emitted by the secondary electromagnetic radiation source 80.

Figure 2a shows behaviour of primary electromagnetic radiation where a skin-print 30 is present while Figure 2b shows behaviour of primary electromagnetic radiation where no skin-print is present. Figure 2c shows behaviour of secondary electromagnetic radiation regardless of whether or not a skin-print is present.

The first end 12 of the translucent waveguide 10 comprises a fingerprint receiving region 20 on a first (upper) surface 16 of the translucent waveguide 10. The fingerprint receiving region 20 may be identified on the first surface 16 by virtue of one or more visible indications on or surrounding the fingerprint receiving region 20. Alternatively, the fingerprint receiving region 20 may be identified by a window bounded by a frame that obscures parts of the first surface 16 that do not form part of the fingerprint receiving region 20. The fingerprint receiving region 20 may be identified by other means.

In the illustrated embodiment, the primary electromagnetic radiation source 40 is configured to emit light towards a surface of the translucent waveguide 10 opposite the fingerprint receiving region 20. Depending on the angle of incidence of the primary electromagnetic radiation relative to the translucent waveguide 10, it may be that the first end 12 of the translucent waveguide 10 also comprises a grating coupler 17 for coupling the primary electromagnetic radiation into the translucent waveguide 10.

A surface of the translucent waveguide 10 in the vicinity of the fingerprint receiving region 20 may serve as a waveguide interface 18 through which electromagnetic radiation may be transmitted or in which electromagnetic radiation may be reflected, dependent upon circumstances. The waveguide interface 18 may or may not be different in surface properties when compared to a surface of the translucent waveguide 10 that surrounds the waveguide interface 18.

The first electromagnetic radiation source 40 is located towards the first end 12 of the translucent waveguide 10 and positioned so as to emit electromagnetic radiation towards the fingerprint receiving region 20 in such a way that the presence or absence of a skin-print in the fingerprint receiving region 20 influences transmission/reflection of the electromagnetic radiation at the waveguide interface 18. The angle of the first electromagnetic radiation source 40 relative to the plane of the waveguide interface 18 may be such that electromagnetic radiation that is coupled into the translucent waveguide 10 propagates along the translucent waveguide 10 in a direction towards the second end 14 of the translucent waveguide 10 and propagates therein by total internal reflection.

The second electromagnetic radiation source 80 is optically coupled to the translucent waveguide 10 towards the second end 14 such that electromagnetic radiation 75 emitted by the second electromagnetic radiation source 80 enters into the translucent waveguide 10 at an angle such that the electromagnetic radiation 75 passes directly through the optical waveguide regardless of whether a skin-print is present. In the illustrated embodiment of Figure 2, while the primary electromagnetic radiation 70 is configured to travel along a the substrate from the first end 12 towards the second end 14, the secondary electromagnetic radiation 75 is directed to travel in a direction largely perpendicular to the primary radiation 70. Optical coupling of the second electromagnetic radiation source 80 to the translucent waveguide 10 may take any appropriate form.

The first photodiode 50 is located so as to detect electromagnetic radiation that is transmitted out of the second end 14 of the translucent waveguide 10. The second photodiode 60 is located so as to detect electromagnetic radiation that is transmitted across the second end 14 of the translucent waveguide 10, as shown in Figure 2c.

While in Figure 2a (and Figure 2c) a skin-print 30 is shown in situ on the skin-print receiving region 20, in Figure 2b no skin-print is present on the skin-print receiving region 20. A comparison between Figures 2a and 2b illustrates how behaviour of electromagnetic radiation 70 emitted by the first electromagnetic radiation source 40 is influenced by the presence or absence of a skin-print 30 on the skin-print receiving region 20.

In the case that no skin-print is present on the skin-print receiving region 20, as is evident from Figure 2b, electromagnetic radiation emitted by the first electromagnetic radiation source 40 is not coupled into the translucent waveguide 10 since it reflects off the fingerprint receiving region 20 and away from the device entirely. Therefore this electromagnetic radiation 70 does not reach the photodiode 50 that is configured to receive electromagnetic radiation that exits the second end 14 of the translucent waveguide 10.

By contrast, as can be seen from Figure 2a, in the case that a skin-print 30 is present on the skin-print receiving region 20, at least a portion of the electromagnetic radiation 70 that arrives at the skin-print receiving region 20 is transmitted into of the translucent waveguide 10 at the waveguide interface 18 by virtue of the presence of the skin-print 30. This is because the waveguide interface 18 is (at least partially) covered by residue of the constituents of the skin-print, hereafter for brevity referred to simply as the skin-print 30. Therefore, instead of the electromagnetic radiation 70 first impacting the fingerprint receiving region 20 of the waveguide interface 18, instead it first impacts the skin-print 30. The relative refractive indices of the translucent waveguide 10 and the skin-print 30 causes refraction of the electromagnetic radiation 70 which means that the angle at which the electromagnetic radiation 70 arrives at the fingerprint receiving region 20 of the translucent waveguide 10 is such that the electromagnetic radiation 70 is coupled into the translucent waveguide 10. Once coupled into the translucent waveguide 10 the angle of the radiation is such that it is totally internally reflected within the translucent waveguide 10 and thereby propagates along the translucent waveguide 10 towards the second end 14.

In very general terms, the greater the (influence of) the skin-print, the greater the amount of primary radiation detected at the first photodetector 50. Where no skin-print is present, little or no electromagnetic radiation 70 from the first electromagnetic radiation source 40 will arrive at and be detected by the photodiode 50. Where a well-defined, strong skin-print is present, a significant proportion of the electromagnetic radiation 70 from the first electromagnetic radiation source 40 will be coupled into the waveguide interface and will arrive at and be detected by the photodiode 50.

By having two electromagnetic radiation sources 40, 80 and two photodiodes 50, 60 wherein only one radiation source is affected by the presence or absence of a skinprint, the amount of secondary radiation detected at the second photodiode 60 can be used as a reference with which the amount of primary radiation detected at the first photodiode 50 can be compared. This allows for elimination of noise within the substrate.

This calculation provides a numeric assessment of the quantity and/or extent of skin-print 30 on the skin-print receiving region 20.

It may be that the first electromagnetic radiation source 40 is configured to emit electromagnetic radiation having a first wavelength or range of wavelengths and that the second electromagnetic radiation source 80 is configured to emit electromagnetic radiation having a second wavelength or range of wavelengths that may or may not be different to the first wavelength or range of wavelengths.

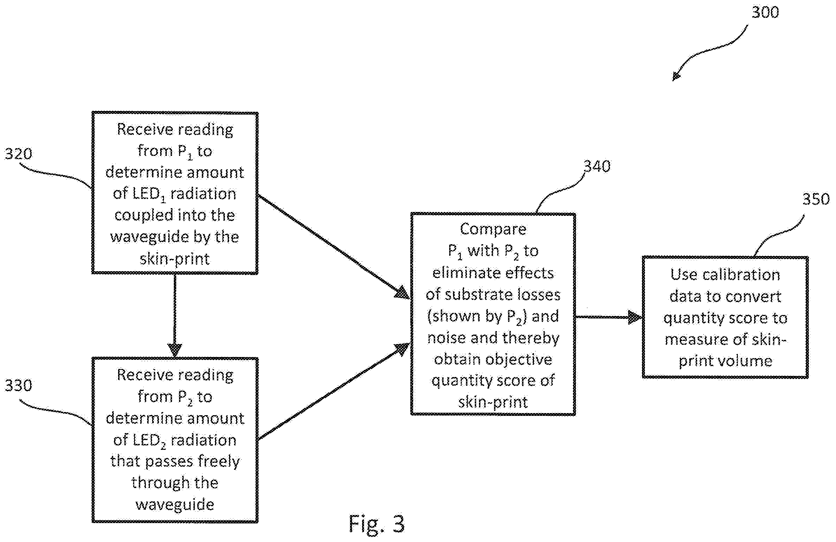

An example of the data processing that may be conducted using the apparatus of the embodiment of Figures 2a, 2b and 2c is shown in the flow chart of Figure 3. In particular, photodiode readings are obtained for the first and second photodiodes P₁ and P₂(320, 330).Processing of these data at a further step 340 allows the effects of losses and noise in the substrate (as evidenced by P₂) to subtracted from the P₁ data in order for the quantity score to be obtained. Through a conversion involving the use of calibration data, a measure of skin-print volume is obtained. (In other embodiments, this may instead be a measure of skin-print mass.) A more detailed discussion of the source of the calibration data is provided below.

It is the fact that the electromagnetic coupling through the waveguide interface 18 is influenced by the quantity of skin-print present that allows for an objective measure of the quantity of a skin-print to be provided by the technique. In brief, the greater the quantity of the skin-print, the greater is the coupling of the electromagnetic radiation 70 from the first electromagnetic radiation source 40. Indeed, it has been established by the Applicant that the amount of primary electromagnetic radiation detected at the first photodiode 50 correlates closely with the volume of the skinprint. The accuracy of this correlation increases when accounting for noise and losses in the substrate which are provided by the secondary electromagnetic radiation detected at the second photodiode 60 which is independent of the volume (or indeed the presence or absence of a skinprint).

It should be noted that Figures 2a, 2b and 2c are highly schematic. As the skilled person readily understands, the electromagnetic radiation 70, 75 will not all travel in exactly the directions indicated by the arrows in Figures 2a, 2b and 2c. Figures 2a, 2b and 2c are intended to illustrate the basic principles on which the technique relies.

In Figures 2a and 2b, the schematic representation of a skin-print 30 (where present) is such as to suggest that it is manifested as a single dome-shaped form on the skin-print receiving region 20. It is emphasised that this representation is highly schematic. In particular, the nature of a skin-print is such that its surface area is of the order of roughly 1 cm to 10 cm while the height is of the order of the order of roughly 0 µm to 10 µm. A schematic representation of a top down view of a skin-print is shown in Figure 2c. Again as the skilled person readily appreciates, the form of skin-prints varies significantly depending upon many factors including the amount of eccrine sweat on the surface of the skin when printed and the force with which a user places the skin against the skin-print receiving region 20 when providing a skin-print. In reality, the skin-print is likely to comprise a number of peaks and troughs, all of which may influence the behaviour of electromagnetic radiation incident upon it in a variety of ways. The peaks may contain sebaceous sweat as well as eccrine sweat which may differently influence the behaviour of the electromagnetic radiation.

The apparatus shown in Figures 2a, 2b and 2c may further comprise optical imaging capability (not shown). The optical imaging capability may be employed to provide an optical image of the skin-print that might be compared with a database of skin-print images, so as to confirm identity of a skin-print.

### Calibration of optical analysis results using other techniques

In order to confirm an objective relationship between the quantity scores obtained by indirect techniques for obtaining mass and/or volume of skin-print (such as the optical technique described above that falls within the scope of the claims) and the actual quantity of the skin-print sample, the Applicant has calibrated its quantity scores using other techniques for determining skin-print quantity and hence skin-print quantity scores.

In one exemplary approach, white light interferometry has been used to obtain a surface profile of a skin-print. White light interferometry is a known technique for providing nanometre-accurate three-dimensional surface profile maps of substrates in the nanometre to centimetre surface area range. By obtaining a 3D surface map of the substrate, the volume of the deposited skin-print can be calculated by performing a three-dimensional integration of the volume beneath the surface profile of the deposited skin-print in order to obtain a volume of deposited skin-print, typically in nanolitres.

In this way, the Applicant has identified that a skin-print quantity score obtained using the optical analysis technique detailed above (and using the apparatus shown in Figures 2a, 2b and 2c) has a linear correlation with the volume of skin-print as measured using the white-light interferometry approach to obtaining a three-dimensional profile of the print and integrating the volume under the surface profile.

In particular, the Applicant has identified over a wide population a linear correlation between the extent to which electromagnetic radiation is coupled by an untreated latent residual skin-print into an optical waveguide and the volume of the said skin-print.

The correlation is such that the data provided by the optical analysis technique provide a value for the volume of skin-print present, within a small and defined level of uncertainty.

### Potential applications of the method

The method of the present disclosure is applicable to a wide range of applications and scenarios. A number of exemplary applications are discussed further below.

One potential application for the method of the present disclosure may be simply to provide evidence that a sufficient volume of skin-print sample is provided in order for a reliable chemical analysis to be conducted. In this way, where an insufficient volume of skin-print has been provided (e.g. where the provider of a sample has sought to evade a test), it may be possible to reject the skin-print sample for chemical analysis in order (a) to avoid cost and (b) instead require the provider of the skin-print sample to provide another sample.

Another potential application for the method of the present disclosure may be to use the quantity test score together with the chemical test result to calculate an amount of a particular analyte of interest per unit of sample. For example, a result may be given in terms of volume of analyte per unit volume of sample, or mass of analyte per unit mass of sample, or mass of analyte per unit volume of sample, or volume of analyte per unit mass of sample, thereby providing a quantitative output.

In this way, it is possible to provide a measure using the same units as are commonly used in the analysis of oral fluid, urine or blood. As the skilled person will appreciate, the unit analyte per unit sample values will not be the same for any analyte. Indeed, for a particular individual, giving simultaneous samples of oral fluid, blood, urine and skin-print, the ratio of analyte to sample may be very different. While these measures are not fully interchangeable, they may be related.

The method may be applied in the context of providing confirmation that an individual is complying with a pharmaceutical or therapeutic treatment regime. In particular, the technique may be used to send data to for example a physician or a prescribing authority which might use the data to satisfy itself that the pharmaceutical or therapeutic treatment regime was being complied with prior to prescribing a future dose of the drug in question.

In a variation on the application of the method in a pharmaceutical or therapeutic treatment regime, the method may have applications in drug trials. Participants in a drug trial may be required to provide skin-print samples at a frequency of up to several times a day without the inconvenience that might result from the need to provide blood, urine or oral fluid samples. The skin-prints may be analysed immediately in the field and the data obtained thereby may be transmitted to the entity running the drug trail in real time in order to obtain granular data regarding the effect of the drug under test on the analytes in the skin-print.

A wide variety of further applications is envisaged and falls within the scope of the appended claims.

### Terminology

In the context of the present disclosure, the term skin-print is used to refer to a skin-print that is latent and/or residual. That is to say the skin-print is what is left behind on a surface once the human skin, from which the skin-print is derived, has been removed. The term skin-print is independent of the size and geometry of the substrate and/or the typical area of contact. As the skilled person would readily appreciate, for the purposes of chemical analysis of the skin-print it is important that the skin-print is not diluted for example by such material as ink which, while may assist for the purpose of visualising the skin-print, may compromise the chemical analysis.

## Claims

1. A method of analysing a skin-print, the method comprising:
receiving a skin-print on a substrate;
performing a quantity test to obtain a quantity score for the skin-print, wherein performing the quantity test to obtain a quantity score for the skin-print comprises determining a volume of skin-print received on the substrate using an optical technique comprising emitting incident light onto the skin-print in order to determine a proportion of incident light that is optically influenced by the skin-print as a proportion of a total of the incident light;
performing a chemical analysis of the skin-print to detect for the presence of one or more chemical species and thereby provide a chemical test result;
storing or transmitting the chemical test result together with the quantity score to enable calculation of a contextualised chemical test result relative to the volume of skin-print.

2. The method of claim 1 wherein the step of determining the volume of skin-print received on the substrate involves measuring one or more attributes of the skin-print received on the substrate and using the one or more measured attributes to calculate the volume of the skin-print.

3. The method of any preceding claim wherein performing the quantity test to obtain a quantity score for the skin-print comprises obtaining an image of the skin-print.

4. The method of any preceding claim further comprising the step of calibrating the quantity test using a secondary quantity test.

5. The method of claim 4 wherein the quantity test comprises measuring influence of a skin-print on the substrate on optical transmission into and/or out of a substrate; and
wherein the secondary quantity test comprises performing white light interferometry on at least a portion of the skin-print in order to produce a topographical representation of the skin-print from which skin-print volume is determined.

6. The method of any preceding claim wherein the chemical analysis comprises one or more of:
mass spectrometry;
paper spray mass spectrometry; and
dissolving the skin-print sample in a solvent.

7. The method of any preceding claim wherein the chemical analysis comprises a lateral flow assay technique.

8. The method of any preceding claim further comprising using the quantity score to scale the chemical test result and:
using the chemical test result together with the quantity score in order to calculate a ratio of at least one analtye present in the chemical test result to skin-print quantity.

9. The method of any preceding claim wherein the substrate is associated with a unique identification feature comprising a unique identifier, wherein the unique identification feature comprises one or more of an RFID tag; a QR code; a bar code; an electronic tag.

10. The method of claim 9 wherein a result of the quantity test is transmitted to a server together with the unique identifier and/or
a result of the chemical analysis is transmitted to a server together with the unique identifier.

11. The method of claim 9 or claim 10 when dependent upon claim 8 wherein the step of using the quantity score to scale the chemical test result is performed on the server.

12. The method of any preceding claim further comprising producing a result in the form of mass of particular chemical constituent per unit volume of skin-print.

13. The method of any preceding claim wherein performing the quantity test to obtain a quantity score for the skin-print involves use of one or more of the following techniques:
Interferometry;
White light interferometry;
Detecting the influence on passage of electromagnetic radiation;
Surface plasmon resonance imaging;
Optical imaging and software processing;
Optical coherence tomography;
Confocal microscopy;
Atomic force microscopy;
3D laser mapping;
Ellipsometry;
Scanning tunnelling microscopy; and
Image analysis following staining with a developer agent such as Ninhydrin and/or
wherein performing analysis of the skin-print to detect for the presence of one or more chemical species and thereby provide a chemical test result involves use of one or more of the following techniques:
mass spectrometry, such as:
liquid chromatography-tandem mass spectrometry (LC-MS-MS);
gas chromatography-tandem mass spectrometry (GC-MS-MS);
matrix assisted laser desorption/ionisation time of flight mass spectrometry (MALDI-TOF MS);
positional mapping mass spectrometry;
paper spray mass spectrometry;
Raman spectroscopy;
chromatography techniques including thin layer chromatography;
lateral flow analysis techniques; and
skin-print development techniques including those employing substances such as Ninhydrin.

14. A method of analysing a skin-print, the method comprising:
receiving a skin-print on a substrate;
performing a quantity test to obtain a quantity score for the skin-print, wherein performing the quantity test to obtain a quantity score for the skin-print comprises determining a volume of skin-print received on the substrate using an optical technique comprising emitting incident light onto the skin-print in order to determine a proportion of incident light that is optically influenced by the skin-print as a proportion of a total of the incident light;
checking that the quantity score meets a pre-defined threshold; and
in cases where the quantity score meets a pre-defined threshold:
performing a chemical analysis of the skin-print to detect for the presence of one or more chemical species and thereby provide a chemical test result; and
storing or transmitting the chemical test result together with the quantity score to enable calculation of a contextualised chemical test result relative to the volume of skin-print.

## Patentansprüche

1. Verfahren zum Analysieren eines Hautabdrucks, wobei das Verfahren umfasst:
Empfangen eines Hautabdrucks an einem Substrat;
Durchführen eines Quantitätstests, um einen Quantitäts-Score für den Hautabdruck zu erhalten, wobei Durchführen des Quantitätstests, um einen Quantitäts-Score für den Hautabdruck zu erhalten, Bestimmen eines Volumens an Hautabdruck, welcher an dem Substrat empfangen wurde, unter Verwendung einer optischen Technik umfasst, welche Aussenden von einfallendem Licht auf den Hautabdruck umfasst, um einen Anteil des einfallenden Lichts, welcher optisch durch den Hautabdruck beeinflusst wird, als einen Anteil einer Gesamtheit des einfallenden Lichts zu bestimmen;
Durchführen einer chemischen Analyse des Hautabdrucks, um das Vorhandensein einer oder mehrerer chemischer Spezies nachzuweisen und dadurch ein chemisches Testergebnis bereitzustellen;
Speichern oder Übertragen des chemischen Testergebnisses zusammen mit dem Quantitäts-Score, um Berechnung eines kontextbezogenen chemischen Testergebnisses relativ zu dem Volumen an Hautabdruck zu ermöglichen.

2. Verfahren nach Anspruch 1, wobei der Schritt des Bestimmens des Volumens an Hautabdruck, welcher an dem Substrat empfangen wurde, Messen eines oder mehrerer Attribute des an dem Substrat empfangenen Hautabdrucks und Verwenden des einen oder der mehreren gemessenen Attribute zum Berechnen des Volumens des Hautabdrucks umfasst.

3. Verfahren nach einem der vorhergehenden Ansprüche, wobei Durchführen des Quantitätstests, um einen Quantitäts-Score für den Hautabdruck zu erhalten, Erhalten eines Bildes des Hautabdrucks umfasst.

4. Verfahren nach einem der vorhergehenden Ansprüche, ferner umfassend den Schritt des Kalibrierens des Quantitätstests unter Verwendung eines sekundären Quantitätstests.

5. Verfahren nach Anspruch 4, wobei der Quantitätstest Messen von Einfluss eines Hautabdrucks an dem Substrat auf optische Transmission in ein und/oder aus einem Substrat umfasst; und
wobei der sekundäre Quantitätstest Durchführen von Weißlichtinterferometrie an wenigstens einem Teil des Hautabdrucks umfasst, um eine topografische Darstellung des Hautabdrucks zu produzieren, aus welcher das Hautabdrucksvolumen bestimmt wird.

6. Verfahren nach einem der vorhergehenden Ansprüche, wobei die chemische Analyse eines oder mehr umfasst aus:
Massenspektrometrie;
Papierspray-Massenspektrometrie; und
Auflösen der Hautabdruckprobe in einem Lösungsmittel.

7. Verfahren nach einem der vorhergehenden Ansprüche, wobei die chemische Analyse eine Lateral-Flow-Assay-Technik umfasst.

8. Verfahren nach einem der vorhergehenden Ansprüche, ferner umfassend Verwendung des Quantitäts-Scores zum Skalieren des chemischen Testergebnisses und:
Verwenden des chemischen Testergebnisses zusammen mit dem Quantitäts-Score, um ein Verhältnis von wenigstens einem Analyten, welcher in dem chemischen Testergebnis vorhanden ist, zu Hautabdruckquantität zu berechnen.

9. Verfahren nach einem der vorhergehenden Ansprüche, wobei das Substrat mit einem eindeutigen Identifikationsmerkmal assoziiert ist, welches eine eindeutige Kennung umfasst, wobei das eindeutige Identifikationsmerkmal eines oder mehrere aus einem RFID-Tag, einem QR-Code, einem Barcode, einem elektronischen Tag umfasst.

10. Verfahren nach Anspruch 9, wobei ein Ergebnis des Quantitätstests zusammen mit der eindeutigen Kennung an einen Server übertragen wird und/oder
ein Ergebnis der chemischen Analyse zusammen mit der eindeutigen Kennung an einen Server übertragen wird.

11. Verfahren nach Anspruch 9 oder Anspruch 10, wenn sie von Anspruch 8 abhängig sind, wobei der Schritt des Verwendens des Quantitäts-Scores, um das chemische Testergebnis zu skalieren, auf dem Server durchgeführt wird.

12. Verfahren nach einem der vorhergehenden Ansprüche, ferner umfassend Produzieren eines Ergebnisses in der Form von Masse eines bestimmten chemischen Konstituenten pro Hautabdruck-Volumeneinheit.

13. Verfahren nach einem der vorhergehenden Ansprüche, wobei Durchführen des Quantitätstests zum Erhalten eines Quantitäts-Scores für den Hautabdruck Verwendung von einer oder mehrerer der folgenden Techniken umfasst:
Interferometrie;
Weißlichtinterferometrie;
Nachweisen des Einflusses auf Passieren elektromagnetischer Strahlung;
Oberflächenplasmonenresonanz-Bildgebung;
Optische Bildgebung und Softwareverarbeitung;
Optische Kohärenztomographie;
Konfokalmikroskopie;
Rasterkraftmikroskopie;
3D-Laser-Mapping;
Ellipsometrie;
Rastertunnelmikroskopie; und
Bildanalyse nach Färben mit einem Entwicklermittel wie Ninhydrin und/oder
wobei Durchführen von Analyse des Hautabdrucks, um das Vorhandensein einer oder mehrerer chemischer Spezies nachzuweisen und dadurch ein chemisches Testergebnis bereitzustellen, Verwendung von einer oder mehrerer der folgenden Techniken umfasst:
Massenspektrometrie, wie:
Flüssigchromatographie-Tandem-Massenspektrometrie (LC-MS-MS);
Gaschromatographie-Tandem-Massenspektrometrie (GC-MS-MS);
Matrix-Assistierte Laser-Desorption/Ionisierung-Flugzeit-Massenspektrometrie (MALDI-TOF MS);
Positionskartierungs-Massenspektrometrie;
Papierspray-Massenspektrometrie;
Raman-Spektroskopie;
Chromatographietechniken einschließlich Dünnschichtchromatographie;
Lateral-Flow-Analysetechniken; und
Hautabdrucks-Entwicklungstechniken einschließlich diejenigen, welche Substanzen wie Ninhydrin nutzen.

14. Verfahren zum Analysieren eines Hautabdrucks, wobei das Verfahren umfasst:
Empfangen eines Hautabdrucks an einem Substrat;
Durchführen eines Quantitätstests, um einen Quantitäts-Score für den Hautabdruck zu erhalten, wobei Durchführen des Quantitätstests, um einen Quantitäts-Score für den Hautabdruck zu erhalten, Bestimmen eines Volumens an Hautabdruck, welcher an dem Substrat empfangen wurde, unter Verwendung einer optischen Technik umfasst, welche Aussenden von einfallendem Licht auf den Hautabdruck umfasst, um einen Anteil des einfallenden Lichts, welcher optisch durch den Hautabdruck beeinflusst wird, als einen Anteil einer Gesamtheit des einfallenden Lichts zu bestimmen;
Prüfen, dass der Quantitäts-Score einen vordefinierten Schwellenwert erfüllt; und
in Fällen, in welchen der Quantitäts-Score einen vordefinierten Schwellenwert erfüllt:
Durchführen einer chemischen Analyse des Hautabdrucks, um das Vorhandensein einer oder mehrerer chemischer Spezies nachzuweisen und dadurch ein chemisches Testergebnis bereitzustellen; und
Speichern oder Übertragen des chemischen Testergebnisses zusammen mit dem Quantitäts-Score, um Berechnung eines kontextbezogenen chemischen Testergebnisses relativ zu dem Volumen an Hautabdruck zu ermöglichen.

## Revendications

1. Procédé d'analyse d'une empreinte cutanée, le procédé comportant :
la réception d'une empreinte cutanée sur un substrat ;
la réalisation d'un test de quantité pour obtenir un score de quantité pour l'empreinte cutanée, dans lequel la réalisation du test de quantité pour obtenir un score de quantité pour l'empreinte cutanée comporte la détermination d'un volume d'empreinte cutanée reçue sur le substrat à l'aide d'une technique optique comportant l'émission de lumière incidente sur l'empreinte cutanée afin de déterminer une proportion de lumière incidente qui est influencée optiquement par l'empreinte cutanée en tant que proportion d'un total de la lumière incidente ;
la réalisation d'une analyse chimique de l'empreinte cutanée pour détecter la présence d'une ou plusieurs espèces chimiques et fournir ainsi un résultat de test chimique ;
le stockage ou la transmission du résultat de test chimique avec le score de quantité pour permettre le calcul d'un résultat de test chimique contextualisé par rapport au volume d'empreinte cutanée.

2. Procédé selon la revendication 1, dans lequel l'étape de détermination du volume d'empreinte cutanée reçue sur le substrat consiste à mesurer un ou plusieurs attributs de l'empreinte cutanée reçue sur le substrat et à utiliser les un ou plusieurs attributs mesurés pour calculer le volume de l'empreinte cutanée.

3. Procédé selon l'une quelconque des revendications précédentes dans lequel la réalisation du test de quantité pour obtenir un score de quantité pour l'empreinte cutanée comporte l'obtention d'une image de l'empreinte cutanée.

4. Procédé selon l'une quelconque des revendications précédentes comportant en outre l'étape d'étalonnage du test de quantité à l'aide d'un test de quantité secondaire.

5. Procédé selon la revendication 4, dans lequel le test de quantité comporte la mesure de l'influence d'une empreinte cutanée sur le substrat sur la transmission optique dans et/ou hors d'un substrat ; et
dans lequel le test de quantité secondaire comporte la réalisation d'une interférométrie à lumière blanche sur au moins une partie de l'empreinte cutanée afin de produire une représentation topographique de l'empreinte cutanée à partir de laquelle le volume d'empreinte cutanée est déterminé.

6. Procédé selon l'une quelconque des revendications précédentes, dans lequel l'analyse chimique comporte une ou plusieurs parmi :
une spectrométrie de masse ;
une spectrométrie de masse par pulvérisation de papier ; et
une dissolution de l'échantillon d'empreinte cutanée dans un solvant.

7. Procédé selon l'une quelconque des revendications précédentes, dans lequel l'analyse chimique comporte une technique de test à flux latéral.

8. Procédé selon l'une quelconque des revendications précédentes, comportant en outre l'utilisation du score de quantité pour mettre à l'échelle le résultat de test chimique et :
l'utilisation du résultat de test chimique conjointement avec le score de quantité afin de calculer un rapport entre au moins un analyte présent dans le résultat de test chimique et la quantité d'empreinte cutanée.

9. Procédé selon l'une quelconque des revendications précédentes, dans lequel le substrat est associé à une caractéristique d'identification unique comportant un identifiant unique, dans lequel la caractéristique d'identification unique comporte une ou plusieurs parmi une étiquette RFID ; un code QR ; un code-barres ; une étiquette électronique.

10. Procédé selon la revendication 9, dans lequel un résultat du test de quantité est transmis à un serveur avec l'identifiant unique et/ou
un résultat de l'analyse chimique est transmis à un serveur conjointement avec l'identifiant unique.

11. Procédé selon la revendication 9 ou la revendication 10 lorsqu'elle dépend de la revendication 8, dans lequel l'étape d'utilisation du score de quantité pour mettre à l'échelle le résultat de test chimique est effectuée sur le serveur.

12. Procédé selon l'une quelconque des revendications précédentes comportant en outre la production d'un résultat sous forme de masse d'un constituant chimique particulier par unité de volume d'empreinte cutanée.

13. Procédé selon l'une quelconque des revendications précédentes dans lequel la réalisation du test de quantité pour obtenir un score de quantité pour l'empreinte cutanée implique l'utilisation d'une ou plusieurs des techniques suivantes :
interférométrie ;
interférométrie à lumière blanche ;
détection de l'influence sur le passage des rayonnements électromagnétiques ;
imagerie par résonance plasmonique de surface ;
imagerie optique et traitement logiciel ;
tomographie par cohérence optique ;
microscopie confocale ;
microscopie à force atomique ;
cartographie laser 3D ;
ellipsométrie ;
microscopie à effet tunnel à balayage ; et
analyse d'image suite à une coloration avec un agent révélateur tel que la ninhydrine et/ou
dans lequel la réalisation d'une analyse de l'empreinte cutanée pour détecter la présence d'une ou plusieurs espèces chimiques et fournir ainsi un résultat de test chimique implique l'utilisation d'une ou plusieurs des techniques suivantes :
spectrométrie de masse, comme :
chromatographie liquide-spectrométrie de masse en tandem (LC-MS-MS) ;
chromatographie en phase gazeuse-spectrométrie de masse en tandem (GC-MS-MS) ;
spectrométrie de masse à temps de vol par désorption/ionisation laser assistée par matrice (MALDI-TOF MS) ;
spectrométrie de masse à cartographie de position ;
spectrométrie de masse par pulvérisation de papier ;
spectroscopie Raman ;
techniques de chromatographie incluant la chromatographie en couche mince ;
techniques d'analyse de flux latéral ; et
techniques de développement d'empreinte cutanée incluant celles utilisant des substances telles que la ninhydrine.

14. Procédé d'analyse d'une empreinte cutanée, le procédé comportant :
la réception d'une empreinte cutanée sur un substrat ;
la réalisation d'un test de quantité pour obtenir un score de quantité pour l'empreinte cutanée, dans lequel la réalisation du test de quantité pour obtenir un score de quantité pour l'empreinte cutanée comporte la détermination d'un volume d'empreinte cutanée reçue sur le substrat à l'aide d'une technique optique comportant l'émission de lumière incidente sur l'empreinte cutanée afin de déterminer une proportion de lumière incidente qui est influencée optiquement par l'empreinte cutanée en tant que proportion d'un total de la lumière incidente ;
la vérification du fait que le score de quantité atteint un seuil prédéfini ; et
dans les cas où le score de quantité atteint un seuil prédéfini :
la réalisation d'une analyse chimique de l'empreinte cutanée pour détecter la présence d'une ou plusieurs espèces chimiques et fournir ainsi un résultat de test chimique ; et
le stockage ou la transmission du résultat de test chimique avec le score de quantité pour permettre le calcul d'un résultat de test chimique contextualisé par rapport au volume d'empreinte cutanée.
